# EUROPÄISCHE PATENTSCHRIFT

(11) **EP 1 182 972 B1**
(45) Veröffentlichungstag und Bekanntmachung des Hinweises auf die Patenterteilung: **15.10.2003**
(21) Anmeldenummer: 00926626.3
(22) Anmeldetag: 25.05.2000
(51) Int. Cl.: A61B 17/00

(54) **OSTEOSYNTHETISCHE KNOCHENPLATTE**
OSTEOSYNTHETIC BONE PLATE
PLAQUE POUR OSTEOSYNTHESE

(30) Priorität: 25.05.1999 DE 29909025 U
(43) Veröffentlichungstag der Anmeldung: 06.03.2002
(73) Patentinhaber: Medartis AG, 4051 Basel (CH)
(72) Erfinder: PFEFFERLE, Joachim, D-79244 Münstertal (DE); JOOS, Ulrich, D-48147 Münster (DE)
(74) Vertreter: Heinen, Detlef
(86) Internationale Anmeldenummer: CH0000299
(87) Internationale Veröffentlichungsnummer: WO00071031

(56) Entgegenhaltungen:
- EP-A- 0 433 852
- WO-A-98/44849
- DE-A- 2 340 880
- US-A- 4 905 679
- US-A- 5 690 631

## Beschreibung

### Anwendungsgebiet der Erfindung

Die vorliegende Erfindung bezieht sich auf eine osteosynthetische Knochenplatte zur Behandlung von Frakturen, insbesondere zur Rekonstruktion von Unterkiefer-Frakturen. Solche Knochenplatten werden intraoperativ eingesetzt, um Knochenfragmente zusammengefügt zu fixieren. Dies kann nach Unfällen, wo ein Knochen in Knochenfragmente zerbrochen ist, innerhalb einer Osteosynthese erforderlich sein oder innerhalb einer orthognatischen bzw. maxillofacialen Behandlung zur chirurgischen Regelung abnormer Stellungen nach einer Osteotomie und anschliessender Positionskorrektur der Knochenfragmente.

Mit einer derartigen Knochenplatte überbrückt und fixiert man vorrangig zwei Knochenfragmente miteinander, wobei je eine Partie der Knochenplatte mit einem Knochenfragment lösbar verbunden ist. Zur Herstellung der Verbindung zwischen der temporär eingesetzten Knochenplatte und den Knochenfragmenten befinden sich in der Knochenplatte Durchgangslöcher zum Einsetzen von Knochenschrauben, welche in die Knochenfragmente eingreifen. Die Knochenplatten sollen sich an die Knochengeometrie passgerecht anbiegen lassen, aber dennoch eine ausreichende Stabilität gewährleisten. Beide Forderungen stehen sich im Prinzip antagonistisch gegenüber. Ferner sollen die Knochenplatten den Aufbau eines interfragmentären Drucks ermöglichen, was durch die gegenüber liegende Anordnung von sogenannten Kompressionslöchern erreicht wird.

### Stand der Technik

In der DE 23 40 880 A1 wird eine massive, einstreifige Knochenplatte zur Behandlung von Kieferbrüchen offenbart, die die Bruchstelle am Kieferknochen überbrückend auf beide zusammenzufügenden Knochenfragmente aufgeschraubt wird. In beiden Hälften der Knochenplatte sind je zwei zur Plattenmitte und zur Bruchstelle hin orientierte Langlöcher vorhanden. Die Langlöcher besitzen auf der dem Kieferknochen abgewendeten Seite eine Ansenkung mit einem als schiefe Ebene ausgebildeten Schraubensitz. Zumindest je ein Langloch pro Hälfte ist schräg zur Plattenmitte ausgerichtet. Auf der dem Kieferknochen zugewandten Seite besitzt die Knochenplatte mittig einen vorspringenden gekerbten Steg. Durch die Anordnung der Langlöcher und die schiefen Schraubensitze werden beim Anziehen der eingesetzten Knochenschrauben die Knochenfragmente zur Bruchstelle hin komprimiert; der so aufgebaute interfragmentäre Druck bewirkt eine verbesserte Knochenbruchheilung.

Durch ihre Steifheit ist diese Platte aber kaum an die vorhandene Knochengeometrie anpassbar. Eine unzureichend an den Kieferknochen angebogene steife Platte führt jedoch dazu, dass das im Kiefer weniger verankerte Knochenfragment zur Platte hinbewegt wird, also Dislokationen auftreten. Selbst geringe Verwerfungen im Frakturbereich führen aber zum Verlust der interfragmentären Abstützung was in einer grösseren Beweglichkeit im Frakturbereich resultiert. Das simple Lochraster an der Platte erlaubt ausserdem wenig Variabilität für die Befestigung an den Knochenfragmenten. Zum Beispiel auch in PREIN, J. (Hrsg.): Manual of Internal Fixation in the Cranio-Facial Skeleton. Springer-Verlag Berlin u.a. 1998, S. 30, werden einstreifige gerade oder bogenförmige Knochenplatten mit Kompressionslöchern zur Behandlung von frakturierten Unterkiefern gezeigt, die eine Dicke von 1,65mm bzw. 2,0mm aufweisen und für Knochenschrauben mit einem Aussengewindedurchmesser von 2,4mm ausgelegt sind.

Dünnere Knochenplatten, z.B. der Stärke zwischen 0,5mm und 0,9mm, die sich besser anbiegen lassen, sind von kraniofacialen Applikationen bekannt (vgl. bei PREIN, a.a.O., S. 28). Hierfür wurden verschiedene Konfigurationen, z.B. als L-Platte, Y-Platte, T-Platte, H-Platte, X-Platte, Doppel-Y-Platte oder Rahmenplatte, entwickelt. Dazu werden Knochenschrauben mit einem Aussengewindedurchmesser von 1,0mm bis 2,0mm verwendet. Diese Knochenplatten besitzen jedoch keine Kompressionslöcher, sondern nur einfache zylindrische Schraubenlöcher mit Ansenkungen zur partiellen Aufnahme des Schraubenkopfes. Auf das Anbringen von Kompressionslöchern in diesen dünneren Platten hat man verzichtet, da man bisher der Auffassung war, für den Aufbau eines interfrakmentären Drucks sei eine grössere Plattenstärke, z.B. 1,65mm oder 2,0mm erforderlich. Ausserdem wäre es mit der bisher verfügbaren Herstellungstechnik enorm aufwendig, in dünnere Knochenplatten, z.B. mit der Stärke von 1,0mm, Kompressionslöcher einzuarbeiten.

### Aufgabe der Erfindung

Nach dem insoweit bekannten Stand der Technik steht noch keine Knochenplatte zur Verfügung, wo selbst bei absolut korrekter Applikation einer herkömmlichen Platte genügend Stabilität für eine ungestörte Knochenheilung garantiert ist und zur verbesserten Knochenbruchheilung ein interfragmentärer Druck für die dynamische Kompression aufgebaut werden kann. Besondere Anforderungen bestehen z.B. für:
- Frakturen in atrophischen Kiefern;
- instabilen Schräg-Frakturen;
- infizierten Unterkiefer-Frakturen;
- instabilen Kieferwinkel-Frakturen; und für
- Unterkiefer-Frakturen bei nicht-kooperativen Patienten.

Angesichts der aufgezeigten Unvollkommenheiten der bis dato bekannten Knochenplatten liegt der Erfindung die Aufgabe zugrunde, eine Knochenplatte insbesondere für die Behandlung von Unterkiefer-Frakturen zu schaffen, die sich durch leichtere Verformbarkeit gut an die jeweils gegebene Kontur der Knochenfragmente anbiegen lässt, aber dennoch eine sichere lagestabile Fixation der Knochenfragmente gewährleistet. D.h. die Knochenplatte muss einerseits gut verformbar sein und andererseits aber eine ausreichende Steifigkeit besitzen. Überdies soll die Knochenplatte Kompressionslöcher aufweisen, um zur Förderung der Knochenheilung einen interfragmentären Druck - im Sinne einer Kompressions-Osteosynthese - erzeugen zu können. Ferner darf die zu schaffende Knochenplatte den am Unterkiefer austretenden Nerv nicht quetschen und bei Trümmerfrakturen sollen auch kleine Knochenfragmente einzeln an der Knochenplatte fixiert werden können. Schliesslich muss sich die Knochenplatte mit herkömmlichen Knochenschrauben applizieren und auf effiziente Weise in Serie fertigen lassen.

### Übersicht über die Erfindung

Die osteosynthetische Knochenplatte dient zur Behandlung von Frakturen, insbesondere zur Rekonstruktion von Unterkiefer-Frakturen. Mittels herkömmlichen Knochenschrauben, die Schraubenköpfe haben, wird die Platte auf die nach dem Prinzip der Kompressions-Osteosynthese an einem Frakturspalt zusammen zu fügenden Knochenfragmente aufgeschraubt. Die Platte weist langlochförmige Kompressionslöcher mit exzentrischen Ansenkungen auf. An der Platte lassen sich eine Längs- und eine Querachse sowie eine Plattenober- und eine Plattenunterseite definieren, wobei letztere den Knochenfragmenten zugewandt ist. Die Knochenplatte besteht aus einem Kompressionsteil, welches zwei zumindest im wesentlichen parallel zur Längsachse verlaufende Plattenstreben besitzt. Beide Plattenstreben sind durch Brückenstege miteinander verbunden, welche die Längsachse schneiden. Eine Plattenstrebe besitzt zumindest an einer der Aussenflanken des Kompressionsteils ein Auge, dem weitere Augen vorgelagert sein können. Vorzugsweise ist an beiden Aussenflanken ein Auge angeordnet. In den Augen befindet sich jeweils ein Kompressionsloch und zwischen den auf einer Plattenstrebe gelegenen Augen erstreckt sich jeweils ein Verbindungssteg, der die Querachse schneidet.

Die nachstehende Beschreibung bezieht sich auf bevorzugte Ausführungsbeispiele der erfindungsgemässen Knochenplatte: Die Kompressionslöcher haben mit ihrer Längserstreckung eine Orientierung in Richtung der Strebenachse oder nehmen zur Strebenachse hin einen Winkel ‡ 0° ein und weisen an der Plattenoberseite exzentrische Ansenkungen auf. In Verlängerung des Kompressionsteils setzen an die Augen Anschlussstege an, weiche sich auf den Strebenachsen erstrecken. Am Ende der Anschlussstege befindet sich jeweils ein tellerförmiges Plattenglied, denen weitere durch Anschlussstege verbundene Plattenglieder vorgelagert sein können. In den Plattengliedern ist je ein Fixierloch zur Aufnahme einer Knochenschraube vorgesehen.

Die Verbindungsstege zwischen den Augen haben eine grössere Breite als die zu den Plattengliedern führenden Anschlussstege. Auf der Plattenoberseite besitzen die Fixierlöcher eine Ansenkung zur partiell versenkten Aufnahme der Köpfe der Knochenschrauben. Vorzugsweise sind an der Knochenplatte zwei Brückenstege vorgesehen, die zur Querachse symmetrisch oder asymmetrisch verlaufen. Bei einer symmetrischen Anordnung der Brückenstege erstrecken sich beide Brückenstege jeweils lateral von der Aussenflanke eines Auges auf der einen Strebenachse hin zur Aussenflanke des gegenüber liegenden Auges auf der anderen Strebenachse. Bei einer asymmetrischen Anordnung der Brückenstege ist abweichend ein Brückensteg zur Querachse hin versetzt und verläuft von dieser aus betrachtet, vor dem Paar von Augen, die sich auf den beiden Strebenachsen gegenüber liegen.

Die Knochenplatte weist eine Materialstärke im Bereich von 0,5mm bis 1,5mm auf und besteht vorzugsweise aus Titan der Güte Grad 1 oder Grad 2. Die in die Kompressionslöcher und in die Fixierlöcher eingesetzten Knochenschrauben sind zum monokortikalen Verschrauben mit den Knochenfragmenten vorgesehen.

### Kurzbeschreibung der beigefügten Zeichnungen

Es zeigen:
- Figur 1A -: eine symmetrische Knochenplatte mit 4 Kompressionslöchern und 4 zylindrischen Fixierlöchern in Draufsicht;
- Figur 1B -: die Darstellung gemäss Figur 1A als Schnitt auf der Linie A-A;
- Figur 2 -: eine asymmetrische Knochenplatte mit 4 Kompressionslöchern und 4 zylindrischen Fixierlöchern in Draufsicht;
- Figur 3 -: eine symmetrische Knochenplatte mit 4 Kompressionslöchern in Draufsicht;
- Figur 4 -: eine symmetrische Knochenplatte gemäss Figur 1A mit 2 winkling zur Längsachse orientierten Kompressionslöchern in Draufsicht;
- Figur 5A -: die symmetrische Knochenplatte gemäss Figur 3 am Beginn einer Kompressions-Osteosynthese mit offenem Knochenspalt;
- Figur 5B -: die Darstellung gemäss Figur 5A während der Kompressions-Osteosynthese mit geschlossenem Knochenspalt;
- Figur 6A -: die symmetrische Knochenplatte gemäss Figur 3 bei einer Kieferwinkel-Fraktur lateral am Unterkiefer eingesetzt;
- Figur 6B -: die symmetrische Knochenplatte gemäss Figur 1A bei einer Kieferwinkel-Fraktur lateral am Unterkiefer eingesetzt;
- Figur 7A -: die symmetrische Knochenplatte gemäss Figur 1A bei einer Median-Fraktur frontal am Unterkiefer eingesetzt; und
- Figur 7B -: die asymmetrische Knochenplatte gemäss Figur 2 bei einer Paramedian-Fraktur seitlich am Unterkiefer eingesetzt.

### Ausführungsbeispiele

Mit Bezug auf die beiliegenden Zeichnungen erfolgt nachstehend die detaillierte Beschreibung von Ausführungsbeispielen zur erfindungsgemässen osteosynthetischen Knochenplatte.

### Figuren 1A und 1 B

Die zur Längsachse **X** und zur Querachse **Y** symmetrisch aufgebaute Knochenplatte **1** weist zwei zur Längsachse **X** parallel verlaufende und zueinander beabstandete Plattenstreben **2** auf. Die Plattenstreben **2** erstrecken sich auf den Strebenachsen **Z.** Äusserlich endet jede Plattenstrebe **2** mit einem tellerförmigen Plattenglied **3**, in dessen Mitte ein zylindrisches Fixierloch **4** vorgesehen ist, welches auf der Plattenoberseite **5** eine Ansenkung **7** zur partiellen versenkten Aufnahme eines Schraubenkopfes besitzt. An der Plattenunterseite **6**, welche eingesetzt den zu verbindenden Knochenfragmenten zugewandt ist, mündet das Fixierloch **4** zylindrisch. Von den Plattengliedern **3** zur Querachse **Y** hin erstreckt sich auf den Strebenachsen **Z** jeweils ein Anschlusssteg **8**.

Die vier Anschlussstege **8** schliessen jeweils an ein Auge **9** des Kompressionsteils **10** der Knochenplatte **1** an. In jedem Auge **9** ist ein in seiner Kontur konventionelles langlochförmiges Kompressionsloch **11** vorhanden, dessen Längserstreckung auf der Strebenachse Z liegt. Jeweils von seiten der Querachse **Y** besitzt das einzelne Kompressionsloch **11** auf der Plattenoberseite **5** eine Ansenkung **12**, die im Kompressionsloch **11**, mit zunehmender Entfernung von der Querachse **Y** harmonisch ausläuft. Somit wird eine im Kompressionsloch **11** quasi exzentrisch eingesetzte Knochenschraube mit Eindringen ihres Kopfes in das Kompressionsloch **11** auf bekannte Weise zur Querachse **Y** hin gedrängt und nimmt hierbei das angeschraubte Knochenfragment mit. Bei aufeinanderzu laufender Richtung der symmetrisch zur Querachse **Y** positionierten Knochenschrauben werden die zusammenzuführenden Knochenfragmente im Wege der Kompressions-Osteosynthese aneinander gepresst.

Zwischen den beidseits der Querachse **Y** auf einer Plattenstrebe **2** liegenden Augen 9 erstreckt sich ein auf der jeweiligen Strebenachse **Z** laufender Verbindungssteg **13,** welcher beide Augen **9** miteinander verbindet. Die Verbindungsstege **13** weisen gegenüber den Anschlussstegen **8** eine grössere Breite auf und besitzen daher auch eine höhere Steifigkeit. Die beiden Plattenstreben **2** sind durch zwei symmetrisch zur Querachse **Y** liegende Brückenstege **14** miteinander verbunden. Die Brückenstege **14** erstrecken sich jeweils lateral von der Einmündung des Anschlussstegs **8** an ein Auge **9** auf der einen Strebenachse **Z** zur Einmündung des Anschlussstegs **8** am gegenüber liegenden Auge **9** auf der anderen Strebenachse **Z.** Durch die Gestaltung der z.B. 1,0mm starken Knochenplatte **1** aus Titan der Güte Grad 1 oder Grad 2 mit zwei Plattenstreben 2 und den Brückenstegen **14** lässt sich die Knochenplatte **1** passgerecht an die jeweilige Geometrie der Knochenfragmente anbiegen und ist zugleich von ausreichender Steifigkeit zur lagestabilen Fixation der Knochenfragmente.

### Figur 2

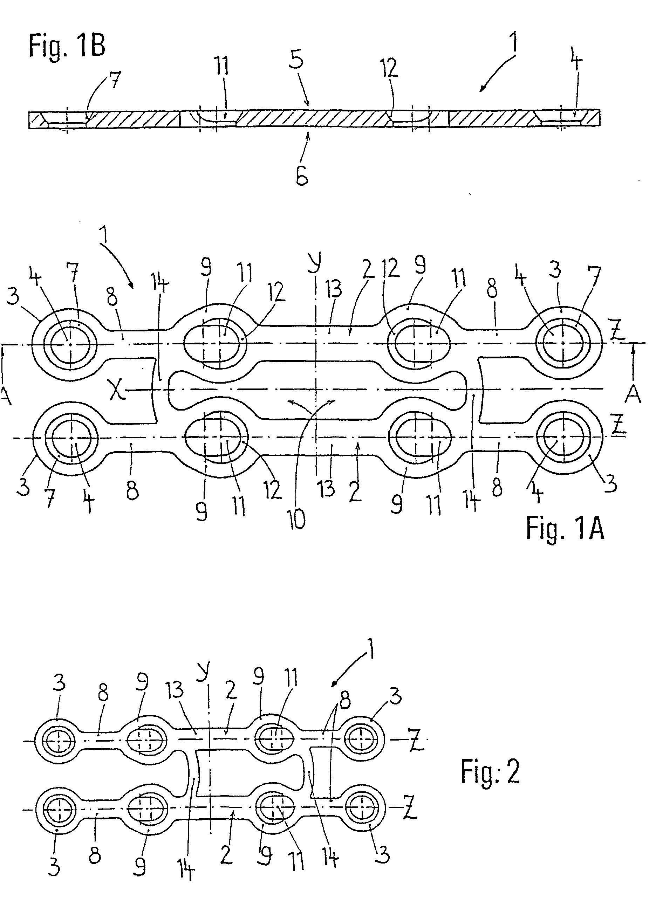

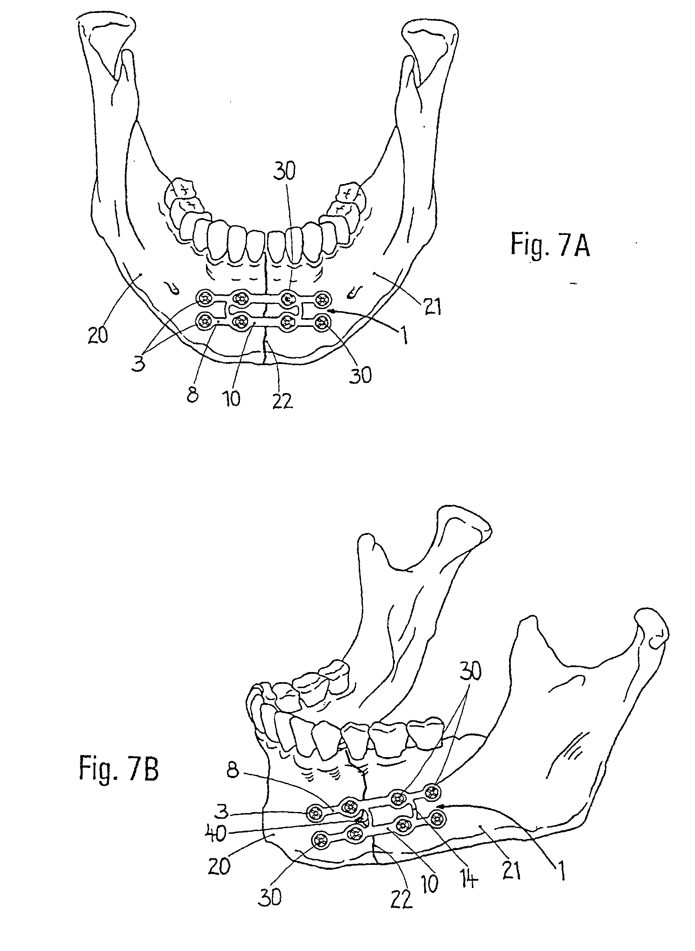

Die Abwandlung gegenüber der Knochenplatte 1 unter Figur **1** besteht darin, dass bei der hier gezeigten Knochenplatte **1** die beide Plattenstreben **2** verbindenden Brückenstege **14** asymmetrisch zur Querachse **Y** angeordnet sind. Der rechte Brückensteg **14** erstreckt sich unverändert lateral von der Einmündung des Anschlussstegs **8** an ein Auge **9** auf der einen Strebenachse **Z** zur Einmündung des Anschlussstegs **8** am gegenüber liegenden Auge **9** auf der anderen Strebenachse **Z**. Der linke Brückensteg **14** hingegen ist zur Querachse **Y** hin versetzt; dieser Brückensteg **14** erstreckt sich lateral von vor der Einmündung des Verbindungsstegs **13** an ein Auge 9 auf der einen Strebenachse Z an eine Position vor die Einmündung des Verbindungsstegs **13** am gegenüber liegenden Auge **9** auf der anderen Strebenachse **Z.** Eine derart konfigurierte Knochenplatte **1** ist vorrangig bei der Applikation für eine Paramedian-Fraktur am Unterkiefer nützlich, wo der austretende Nerv nicht von einem Plattenteil gedrückt werden darf (s. die Beschreibung zur Fig. 7B).

### Figur 3

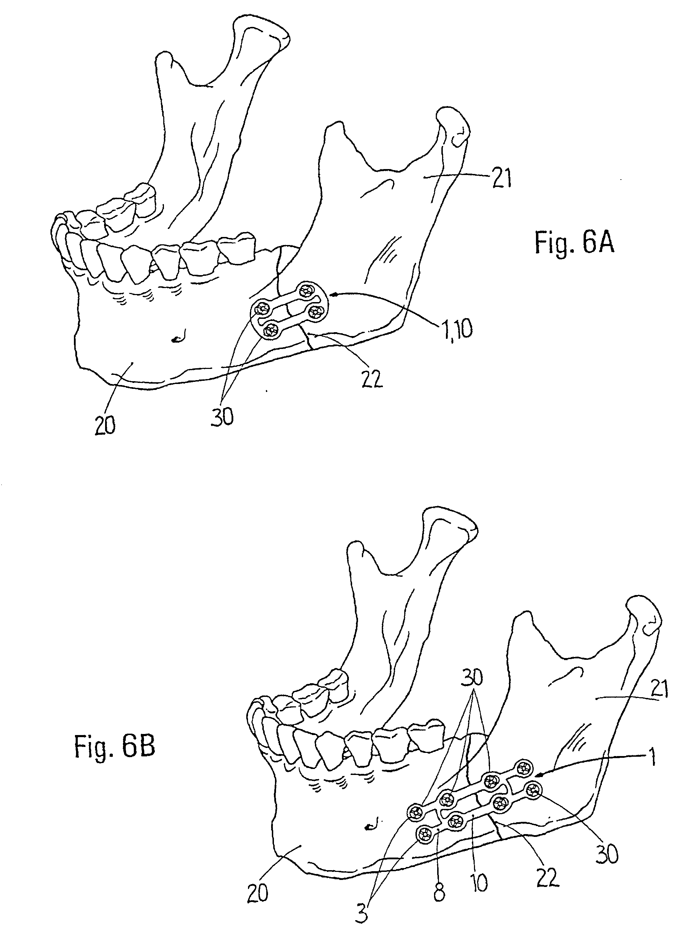

Bei dieser vereinfachten Knochenplatte **1** ist nur das Kompressionsteil **10** vorgesehen; die nach aussen weisenden Anschlussstege **8** und die ganz aussen liegenden Plattenglieder **3** mit den Fixierlöchern **4** sind hier nicht vorhanden. Im Prinzip, wie bei der Ausführungsform gemäss Figur 1, erstrecken sich die Brückenstege **14** jeweils von der Aussenflanke von einem Auge **9** auf der einen Strebenachse **Z** zur Aussenflanke des gegenüber liegenden Auges **9** auf der anderen Strebenachse Z. Eine beispielhafte Applikation dieser Knochenplatte **1** wird bei Fig. 6A beschrieben.

### Figur 4

Die Besonderheit an dieser symmetrischen Ausführungsform gegenüber der Basisvariante gemäss Figur 1 besteht darin, dass die beiden langlochförmigen Kompressionslöcher **11** mit den Augen **9** an der unteren Plattenstrebe **2** in ihrer Längserstreckung nicht auf der Strebenachse **Z** bzw. parallel zur Längsachse **X** verlaufen, sondern den Winkel α einnehmen. Dies ist von Nutzen, wenn bei einem speziellen Frakturverlauf von der unteren Plattenstrebe **2** in schräger Richtung auf die Längsachse **X** zu ein interfragmentärer Druck aufgebaut werden soll.

### Figuren 5A und 5B

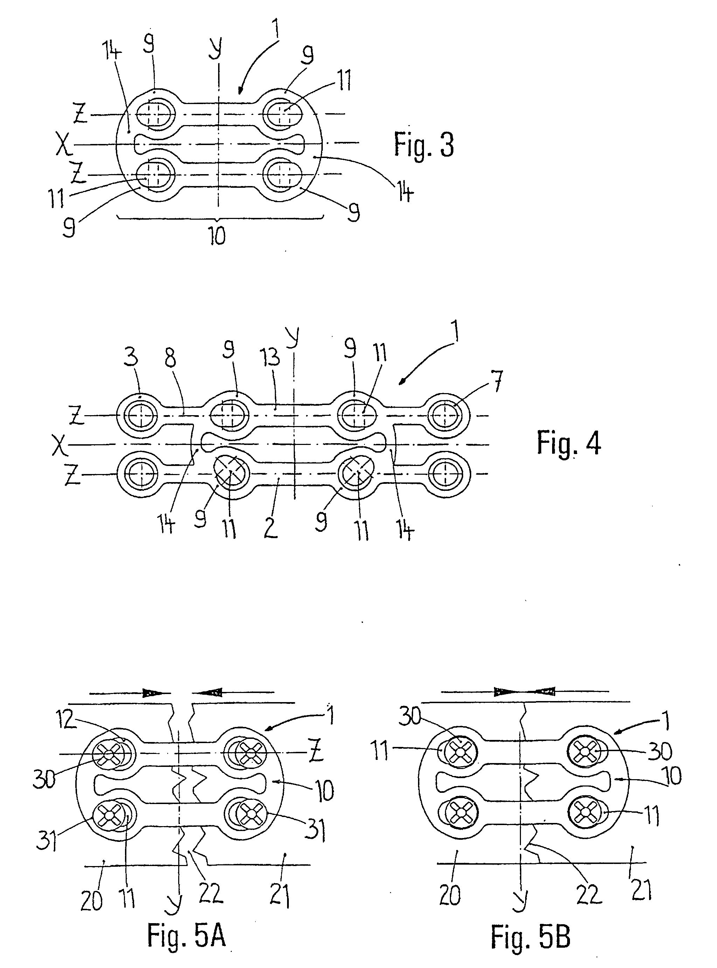

Die beiden Figuren veranschaulichen das Funktionsprinzip der Kompressions-Osteosynthese unter beispielhafter Verwendung einer Knochenplatte **1** gemäss Fig. 3.

In der Ausgangssituation (s. Fig. 5A) besteht zwischen den aneinander zu fügenden Knochenfragmenten **20,21** der zu schliessende Frakturspalt **22**, über den die Knochenplatte **1** mit ihrem Kompressionsteil **10** gelegt ist. Die Knochenplatte **1** ist vom Typ der sogenannten Miniplatten. Die Knochenschrauben **30** werden in den langlochförmigen Kompressionslöchern **11** abgekehrt von den Ansenkungen **12** eingebracht, d.h., betrachtet auf der jeweiligen Plattenstrebe 2 und auf der zugehörigen Strebenachse **Z,** in grösstmöglichem Abstand voneinander. Wie ersichtlich, sind die Schraubenköpfe **31** der in die Knochenfragmente vorzugsweise monokortikal eingeschraubten Knochenschrauben **30**, also vom Frakturspalt **22** und der Querachse **Y** weiter entfernt.

Wie Fig. 5B zeigt, gelangen mit zunehmendem Eindrehen der Knochenschrauben **30** in die Knochenfragmente **20,21** die Schraubenköpfe **31 -** durch die speziell konturierten Ansenkungen **12** in den Kompressionslöchern **11 -** in die Ansenkungen **12**. Hierbei werden die an den Knochenschrauben 30 hängenden Knochenfragmente **20,21** in Richtung der Querachse **Y** und des Frakturspalts **22** mitgenommen bis schliesslich der Frakturspalt **22** geschlossen ist und die Knochenfragmente **20,21** mit Kompression zusammen gefügt sind.

### Figuren 6A bis 7B

Diese Figurenfolge veranschaulicht beispielhaft einige Applikationen der erfindungsgemässen Knochenplatte **1** in unterschiedlicher Ausführungsform bei Unterkiefer-Frakturen.
- Fig. 6A:: Mit einer Knochenplatte **1** in der Konfiguration gemäss Fig. 3, welche nur das Kompressionsteil **10** aufweist, wird eine Kieferwinkel-Fraktur behandelt. Die mit vier Knochenschrauben **30** fixierte Knochenplatte **1** überbrückt den Frakturspalt **22** und verbindet als Knochenfragmente **20,21** den Corpus mandibulae mit dem Ramus mandibulae.
- Fig. 6B:: Mit einer symmetrischen Knochenplatte **1** in der Konfiguration gemäss Fig. 1, welche das Kompressionsteil **10** und die sich darüber hinaus erstreckenden Anschlussstege **8** und die äusseren Plattenglieder **3** umfasst, wird ebenfalls eine Kieferwinkel-Fraktur behandelt. Über den Frakturspalt **22** verbunden sind wiederum als Knochenfragmente **20,21** der Corpus mandibulae mit dem Ramus mandibulae. Zusätzlich zu den vier Knochenschrauben **30** im Kompressionsteil **10** sind die Knochenfragmente **20,21** mit jeweils zwei in den äusseren Plattengliedern **3** eingesetzten Knochenschrauben **30** gesichert.
- Fig. 7A:: Ebenfalls mit einer symmetrischen Knochenplatte **1** in der Konfiguration gemäss Fig. 1, welche das Kompressionsteil **10** und die sich darüber hinaus erstreckenden Anschlussstege **8** und die äusseren Plattenglieder **3** umfasst, wird eine Median-Fraktur am Unterkiefer be- handelt. Über den median liegenden Frakturspalt **22** sind als Knochenfragmente **20,21** zwei Teile des gebrochenen Corpus mandibulae miteinander verbunden. Auch hier sind zusätzlich zu den vier Knochenschrauben **30** im Kompressionsteil **10** die Knochenfragmente **20,21** mit den jeweils zwei in den äusseren Plattengliedern **3** eingesetzten Knochenschrauben **30** lagestabil fixiert.
- Fig. 7B:: Zur gezeigten Behandlung einer Paramedian-Fraktur am Unterkiefer wird eine asymmetrischen Knochenplatte **1** in der Konfiguration gemäss Fig. 2 verwendet. Diese Knochenplatte **1** besteht aus dem Kompressionsteil **10,** den sich darüber hinaus erstreckenden Anschlussstegen **8,** den äusseren Plattengliedern **3** und den asymmetrisch angeordneten Brückenstegen **14** zwischen den beiden Plattenstreben **2**. Mittels der Knochenplatte 1 sind über den paramedian liegenden Frakturspalt **22** als Knochenfragmente **20,21** zwei Teile des aussermittig gebrochenen Corpus mandibulae miteinander verbunden. Der austretende Nerv **40** (Foramen mentale) liegt frei und wird von keinem der Plattenteile gequetscht. Insgesamt sind die Knochenfragmente **20,21** jeweils mit vier Knochenschrauben **30** gesichert.

## Patentansprüche

1. Osteosynthetische Knochenplatte (**1**) zur Behandlung von Frakturen, insbesondere zur Rekonstruktion von Unterkiefer-Frakturen, wobei die Knochenplatte (**1**):
a) dazu bestimmt ist, mittels herkömmlichen Knochenschrauben **(30),** die Schraubenköpfe (**31**) haben, auf die nach dem Prinzip der Kompressions-Osteosynthese an einem Frakturspalt (**22**) zusammen zu fügenden Knochenfragmente **(20,21)** aufgeschraubt zu werden;
b) langlochförmige Kompressionslöcher (**11**) mit exzentrischen Ansenkungen (**12**) aufweist;
c) eine Längsachse (**X**) und eine Querachse (**Y**) besitzt; und
d) eine Plattenoberseite (**5**) und eine den Knochenfragmenten (**20,21**) zugewandte Plattenunterseite (**6**) besitzt, **dadurch gekennzeichnet, dass**
e) die Knochenplatte (**1**) aus einem Kompressionsteil (**10**) besteht, welches zwei zumindest im wesentlichen parallel zur Längsachse (X) verlaufende Plattenstreben (**2**) besitzt;
f) die beiden Plattenstreben (**2**) durch Brückenstege (**14**) miteinander verbunden sind, welche die Längsachse (X) schneiden;
g) eine Plattenstrebe (**2**) *an einer der Aussenflanken oder an beiden Aussenflanken des Kompressionsteils* ***(10)*** ein Auge (**9**) hat, *dem weitere Augen (****9****) vorgelagert sein können;*
*h) sich* in den *Augen (****9****)* jeweils ein Kompressionsloch **(11)** befindet; und
*i)* sich zwischen den *auf einer Plattenstrebe* (**2**) *gelegenen* Augen (**9**) *jeweils* ein Verbindungssteg (**13**) erstreckt, der die Querachse (**Y**) schneidet
j) die Knochenplatte eine Materialstärke im Bereich von 0,5mm bis 1,5mm aufweist.

2. Osteosynthetische Knochenplatte (**1**) nach Anspruch 1, **dadurch gekennzeichnet, dass** die Kompressionslöcher **(11)**
a) mit ihrer Längserstreckung eine Orientierung in Richtung der Strebenachse (**Z**) haben; oder
b) zur Strebenachse (**Z**) einen Winkel (α) ∅ 0° einnehmen; und
c) an der Plattenoberseite (**4**) exzentrische Ansenkungen **(12)** aufweisen.

3. Osteosynthetische Knochenplatte (**1**) nach Anspruch 1 oder 2, dadurch gekenntzeichnet, dass
a) in Verlängerung des Kompressionsteils (**10**) an die Augen (**9**) Anschlussstege (**8**) ansetzen, welche sich auf den Strebenachsen (**Z**) erstrecken;
b) sich am Ende der Anschlussstege (**8**) *jeweils ein* tellerförmiges Plattenglied *(****3****) befindet, denen weitere durch Anschlussstege (****8****) verbundene Plattenglieder* ***(3)*** *vorgelagert sein können;* und
c) in den Plattengliedern (**3**) je ein Fixierloch (**4**) zur Aufnahme einer Knochenschraube (30) vorgesehen ist.

4. Osteosynthetische Knochenplatte (**1**) nach Anspruch 3, **dadurch gekennzeichnet, dass**
a) die Verbindungsstege (**13**) zwischen den Augen (**9**) von grösserer Breite sind als die zu den Plattengliedern (**3**) führenden Anschlussstege (**8**) und;
b) die Fixierlöcher (**4**) auf der Plattenoberseite (**5**) eine Ansenkung (**12**) zur partiell versenkten Aufnahme der Köpfe (**31**) der Knochenschrauben (**30**) haben.

5. Osteosynthetische Knochenplatte (**1**) nach einem,der Ansprüche 1 bis 4, **dadurch gekennzeichnet, dass** zwei Brückenstege (**14**) vorgesehen sind, die sich zur Querachse (**Y**) symmetrisch oder asymmetrisch erstrecken.

6. Osteosynthetische Knochenplatte (**1**) nach Anspruch 5, **dadurch gekennzeichnet, dass**
a) bei einer symmetrischen Anordnung der Brückenstege (**14**) sich beide Brückenstege (**14**) jeweils lateral von der Aussenflanke eines Auges (**9**) auf der einen Strebenachse (**Z**) hin zur Aussenflanke des gegenüber liegenden Auges (**9**) auf der anderen Strebenachse (**Z**) erstrecken; und
b) bei einer asymmetrischen Anordnung der Brückenstege **(14)** abweichend ein Brückensteg (**14**) zur Querachse (**Y**) hin versetzt ist und von dieser aus betrachtet, vor dem Paar von Augen (**9**), die sich auf den beiden Strebenachsen (**Z**) gegenüber liegen, verläuft.

7. Osteosynthetische Knochenplatte (**1**) nach einem der Ansprüche 1 bis 6, **dadurch gekennzeichnet, dass** diese aus Titan, *vorzugsweise der Güte Grad 1 oder Grad 2,* besteht.

8. Osteosynthetische Knochenplatte **(1)** nach einem der Ansprüche **1** bis 7, **dadurch gekennzeichnet, dass** die in die Kompressionslöcher **(11)** und in die Fixierlöcher (**4**) eingesetzten Knochenschrauben (**30**) zum monokortikalen Verschrauben mit den Knochenfragmenten (**20,21**) vorgesehen sind,

## Claims

1. An osteosynthetic bone plate (1) for the treatment of fractures, in particular for the reconstruction of mandibular fractures, in which the bone plate (1):
(a) is intended to be screwed by means of conventional bone screws (30), which have screw heads (31), onto the bone fragments (20, 21) which are to be joined together in accordance with the principle of compression osteosynthesis at a fracture line (22);
(b) has oblong compression holes (11) with eccentric countersinks (12);
(c) has a longitudinal axis (X) and a transverse axis (Y) ; and
(d) has a plate upper side (5) and a plate lower side (6) which faces toward the bone fragments (20, 21), **characterized in that**
(e) the bone plate (1) comprises a compression part (10) which has two plate braces (2) extending at least substantially parallel to the longitudinal axis (X);
(f) the two plate braces (2) are connected to one another by bridging struts (14) which intersect the longitudinal axis (X);
(g) a plate brace (2) has an eyelet (9) on one of the outer flanks or on both outer flanks of the compression part (10), and additional eyelets (9) can be positioned in front of this eyelet;
(h) a compression hole (11) is situated in each of the eyelets (9); and
(i) a connecting strut (13) which intersects the transverse axis (Y) extends between the eyelets (9) located on each plate brace (2);
(j) the bone plate has a material thickness in the range from 0.5 mm to 1.5 mm.

2. The osteosynthetic bone plate (1) as claimed in claim 1, **characterized in that** the compression holes (11)
(a) have, with their longitudinal extent, an orientation in the direction of the brace axis (Z); or
(b) assume an angle (α) ø 0° in relation to the brace axis (Z); and
(c) have eccentric countersinks (12) on the plate upper side (4).

3. The osteosynthetic bone plate (1) as claimed in claim 1 or 2, **characterized in that**
(a) in a continuation of the compression part (10), attachment struts (8) adjoin the eyelets (9) and extend on the brace axes (Z);
(b) a disk-shaped plate member (3) is situated at the end of each of the attachment struts (8), and further plate members (3) connected by attachment struts (8) can be positioned in front of said plate member (3); and
(c) a fixation hole (4) for receiving a bone screw (30) is in each case provided in the plate members (3).

4. The osteosynthetic bone plate (1) as claimed in claim 3, **characterized in that**
(a) the connecting struts (13) between the eyelets (9) have a greater width than the attachment struts (8) leading to the plate members (3); and
(b) the fixation holes (4) on the plate upper side (5) have a countersink (12) for receiving the heads (31) of the bone screws (30) in a partially recessed manner.

5. The osteosynthetic bone plate (1) as claimed in one of claims 1 through 4, **characterized in that** two bridging struts (14) are provided which extend symmetrically or asymmetrically with respect to the transverse axis (Y).

6. The osteosynthetic bone plate (1) as claimed in claim 5, **characterized in that**
(a) in the case of a symmetrical arrangement of the bridging struts (14), both bridging struts (14) extend laterally from the outer flank of one eyelet (9) on one brace axis (Z) to the outer flank of the opposite eyelet (9) on the other brace axis (Z); and
(b) in the case of an asymmetric arrangement of the bridging struts (14), by contrast, one bridging strut (14) is offset toward the transverse axis (Y) and, as seen from the latter, extends in front of the pair of eyelets (9) which lie opposite each other on the two brace axes (Z).

7. The osteosynthetic bone plate (1) as claimed in one of claims 1 through 6, **characterized in that** it is made of titanium, preferably of quality grade 1 or grade 2.

8. The osteosynthetic bone plate (1) as claimed in one of claims 1 through 7, **characterized in that** the bone screws (30) inserted in the compression holes (11) and in the fixation holes (4) are provided for monocortical screwing to the bone fragments (20, 21).

## Revendications

1. Plaque à os (1) d'ostéosynthèse pour le traitement de fractures, notamment pour la reconstruction de fractures de la mâchoire inférieure, dans laquelle ladite plaque à os (1) :
a) est destinée à être vissée sur des fragments d'os (20, 21) à assembler à travers une fente de fracture (22) selon le principe de l'ostéosynthèse par compression au moyen de vis à os conventionnelles (30) qui comportent des têtes de vis (31 );
b) comporte des trous de compression oblongs (11) pourvus de chanfreins excentrés (12) :
c) possède un axe longitudinal (X) et un axe transversal (Y), et
d) présente une face supérieure de plaque (5) et une face inférieure de plaque (6) tournée vers les fragments d'os (20, 21), **caractérisée en ce que**
e) la plaque à os (1) est constituée d'une pièce de compression (10) comprenant deux tirants de plaque (2) qui s'étendent au moins sensiblement parallèlement à l'axe longitudinal (X) ;
f) les deux tirants de plaque (2) sont reliés entre eux par des pontets (14) qui coupent l'axe longitudinal (X) ;
g) un tirant de plaque (2) présente, sur l'un des flancs extérieurs ou sur les deux flancs extérieurs de la pièce de compression (10), un oeillet (9) devant lequel d'autres oeillets (9) peuvent être disposés ;
h) un trou de compression (11) se trouve respectivement dans les oeillets (9) ; et
i) une patte de liaison (13) s'étend entre les oeillets respectifs (9) disposés sur un tirant de plaque (2) et coupe l'axe transversal (Y) ;
j) la plaque à os présente une épaisseur de matière dans la plage de 0,5 mm à 1,5 mm.

2. Plaque à os (1) d'ostéosynthèse selon la revendication 1, **caractérisée en ce que** les trous de compression ( 11 )
a) ont dans leur extension longitudinale une orientation dans la direction de l'axe (Z) de tirant ; ou
b) définissent sur l'axe de tirant (Z) un angle (α) ∅ 0° ; et
c) comportent des chanfreins excentrés (12) sur la face supérieure (4) de la plaque.

3. Plaque à os (1) d'ostéosynthèse selon la revendication 1 ou 2, **caractérisée en ce que**
a) des pattes de jonction (8) sont fixées aux oeillets (9), dans le prolongement de la pièce de compression (10), et s'étendent suivant les axes (Z) de tirants ;
b) un organe de plaque (3) en forme d'assiette se trouve à l'extrémité respective de la patte de jonction (8), devant lequel peuvent être disposés d'autres organes de plaques (3) reliés par des pattes de jonction (8) ; et
c) un trou de fixation (4) est prévu dans chaque organe de plaque (3) pour loger une vis à os (30).

4. Plaque à os (1) d'ostéosynthèse selon la revendication 3, **caractérisée en ce que**
a) entre les oeillets (9), les pattes de liaison (13) ont une largeur plus grande que les pattes de jonction (8) conduisant aux organes de plaque (3) et ;
b) sur la face supérieure (5) de la plaque, les trous de fixation (4) présentent un chanfrein (12) pour noyer partiellement les têtes (31) des vis à os (30).

5. Plaque à os (1) d'ostéosynthèse selon l'une quelconque des revendications 1 à 4, **caractérisée en ce que** sont prévus deux pontets (14) qui s'étendent symétriquement ou asymétriquement par rapport à l'axe transversal (Y).

6. Plaque à os (1) d'ostéosynthèse selon la revendication 5, **caractérisée en ce que**
a) dans un agencement symétrique des pontets (14), les deux pontets (14) s'étendent chacun latéralement au flanc extérieur d'un oeillet (9), en s'éloignant d'un axe de tirant (Z) jusqu'au flanc extérieur de l'oeillet (9) situé en vis-à-vis sur l'autre axe de tirant (Z) ; et
b) dans un agencement asymétrique des pontets (14), un pontet (14) est décalé vers l'axe transversal (Y) et, en regardant à partir de celui-ci, s'étend devant la paire d'oeillets (9) qui sont en vis-à-vis sur les deux axes de tirants (Z).

7. Plaque à os (1) d'ostéosynthèse selon l'une quelconque des revendications 1 à 6, **caractérisée** en ce quelle est constituée en titane, de préférence de la qualité de degré 1 ou de degré 2.

8. Plaque à os (1) d'ostéosynthèse selon l'une quelconque des revendications 1 à 7, **caractérisée en ce que** les vis à os (30) insérées dans les trous de compression (11) et dans les trous de fixation (4) sont prévus pour un vissage monocortical avec les fragments d'os (20, 21).
